# EUROPÄISCHE PATENTSCHRIFT

(11) **EP 0 999 813 B1**
(45) Veröffentlichungstag und Bekanntmachung des Hinweises auf die Patenterteilung: **20.02.2002**
(21) Anmeldenummer: 98938708.9
(22) Anmeldetag: 28.07.1998
(51) Int. Cl.: A61F 13/15

(54) **ABSORBIERENDER HYGIENEARTIKEL ZUM EINMALIGEN GEBRAUCH**
ABSORBENT SINGLE USE HYGIENE ITEM
ARTICLE HYGIENIQUE ABSORBANT A USAGE UNIQUE

(30) Priorität: 29.07.1997 DE 19732550
(43) Veröffentlichungstag der Anmeldung: 17.05.2000
(73) Patentinhaber: Paul Hartmann Aktiengesellschaft, 89522 Heidenheim (DE)
(72) Erfinder: MALOWANIEC, Krzysztof, Daniel, D-89522 Heidenheim (DE)
(74) Vertreter: Friz, Oliver
(86) Internationale Anmeldenummer: EP9804702
(87) Internationale Veröffentlichungsnummer: WO9905999

(56) Entgegenhaltungen:
- EP-A- 0 151 018
- EP-A- 0 689 818
- WO-A-92/11830
- WO-A-95/10996
- GB-A- 2 296 438
- US-A- 4 798 603
- US-A- 5 294 478

## Beschreibung

Die Erfindung betrifft einen absorbierenden Hygieneartikel zum einmaligen Gebrauch mit einem flüssigkeitsdichten Rückblatt, einem flüssigkeitsdurchlässigen Deckblatt, einem dazwischen angeordneten zur Speicherung von Körperflüssigkeiten, insbesondere von Urin, geeigneten superabsorbierende Polymer-Materialien (SAP-Materialien) enthaltenden Saugkörper und einer flüssigkeitsaufnehmenden Zwischenschicht, die aus einer oberen Lage (18) aus synthetischen Fasern und einer unteren Lage (20) besteht, wobei das durchschnittliche Porenvolumen der oberen Lage (18) größer ist als das durchschnittliche Porenvolumen der unteren Lage (20) der Zwischenschicht (8) und wobei die Zwischenschicht zwischen dem Saugkörper (6) und dem Deckblatt (2) angeordnet ist.

Ein derartiger Hygieneartikel ist aus der DE 92 18 991 U1 bekannt. Ferner ist aus der EP 0 689 818 A2 ein Hygieneartikel bekannt, bei dem die Zwischenschicht zusammen mit der Saugschicht aus einem vielschichtigen Flächenmaterial hergestellt ist, welches ebenfalls die Merkmale des Oberbegriffs des Anspruchs 1 aufweist.

Bei Hygieneartikeln, insbesondere Windeln oder Inkontinenzvorlagen, mit SAP-Materialien im Saugkörper besteht die Gefahr des sogenannten Gelblockings. Im Bereich des Flüssigkeitsanfalls quellen die SAP-Materialien, so dass, insbesondere nach mehrfachem Flüssigkeitsanfall in dem in Rede stehenden Bereich die weitere Flüssigkeitsaufnahme behindert ist. Die zwischen Deckblatt und Saugkörper vorgesehene Zwischenschicht soll daher die auftreffende Flüssigkeit als eine Art Zwischenpuffer aufnehmen innerhalb der Zwischenschicht verteilen und in den Saugkörper ableiten. Auf diese Weise kann Flüssigkeit vom Bereich des Flüssigkeitsanfalls weggeleitet und an davon entfernter Stelle, wo der Saugkörper bzw. die darin enthaltenen SAP-Materialien noch "unbenutzt" zur Verfügung stehen, in diesen eingeleitet werden.

Aus der WO 91/11162 ist ein Hygieneartikel bekannt, bei dem oberhalb des Saugkörpers eine vernetzten Cellulosefasern aufweisende Flüssigkeitserfassungs- und verteilungsschicht vorgesehen ist.

Durch die chemische Vernetzung der Cellulosemoleküle, die wohlgemerkt innerhalb der Cellulosefasern durch Vernetzung der Cellulosemoleküle einer Faser und nicht zwischen den Fasern vorkommt, wird erreicht, dass die Fasern in vergleichsweise steifem, bauschelastischem Zustand vorliegen. Das intramolekulare Absorbtionsvermögen der Fasern ist reduziert. Solche Fasern sind besser als die chemisch nicht vernetzten Cellulosefasern des Saugkörpers geeignet, auch wiederholt auftretende Flüssigkeitsschwälle zu verarbeiten, d.h. schnell aufzunehmen und in sich und in den Saugkörper weiter zu leiten. Bei dem bekannten Hygieneartikel enthält die Zwischenschicht neben den chemisch vernetzten Cellulosefasern ein thermoplastisches Bindematerial, in Form von beigemischten thermoplastischen Fasern. Durch Erhitzen des Fasergemischs werden die thermoplastischen Fasern erschmolzen und fixieren so die chemisch vernetzten Cellulosefasern klebstoffartig miteinander, was als besonders vorteilhaft dargestellt wird.

Die EP 0 397 110 A1 beschreibt eine Windel, bei der die Funktion der vorübergehenden Flüssigkeitsaufnahme durch einer auf synthetischen Fasern basierenden, voluminösen Vliesstoffschicht übernommen wird, die wiederum als Zwischenschicht zwischen Saugkörper und Deckblatt angeordnet ist. Um die Funktion der Zwischenspeicherung und Verteilung der anfallenden Flüssigkeit in befriedigender Weise erfüllen zu können, muss diese Schicht aber ein Flächengewicht von wenigstens 60 g/m² aufweisen. Die Materialien zur Bildung der voluminösen Vliesstoffschicht sind teuer, werfen logistische Probleme auf und lassen sich nur sehr schwer in eine schnelllaufende Maschine integrieren, da eine Endlosrolle dieser Materialien bei endlichen, üblicherweise verwendbarem Durchmessern nur eine sehr begrenzte Lauflänge aufweisen kann, so dass ein häufiger Rollenwechsel die Folge ist.

Ausgehend vom vorstehend beschriebenen Stand der Technik liegt der vorliegenden Erfindung die Aufgabe zugrunde, einen Hygieneartikel mit einem hohen Anteil an super-absorbierenden Polymer-Materialien im Saugkörper bereitzustellen, dessen Speicherkapazität noch besser und vollständiger genutzt werden kann, als dies bei dem aus der WO 91/11162 bekannten Hygieneartikel der Fall ist. Der Hygieneartikel soll desweiteren in wirtschaftlicher Weise herstellbar sein. Ausgehend von den gattungsgemäßen Hygieneartikeln liegt der vorliegenden Erfindung die Aufgabe zugrunde, einen einfach aufgebauten und damit auf wirtschaftliche Weise herstellbaren Hygieneartikel zu schaffen, dessen Flüssigkeitsaufnahmeverhalten und dessen Flüssigkeitshaltevermögen, insbesondere nach mehrmaligem Auftreffen von Flüssigkeit, verbessert ist. Es soll ferner verhindert werden, dass nach mehrfachem Flüssigkeitsanfall ein Benutzer des Hygieneprodukts in der Zwischenschicht verbleibende Flüssigkeit etwa durch den Druck des Körpergewichts an der Haut verspürt.

Diese Aufgabe wird bei einem Hygieneartikel der eingangs erwähnten Art erfindungsgemäß durch die Merkmale des kennzeichnenden Teils des Anspruchs 1 gelöst.

Es wird nach der Erfindung vorgeschlagen, die untere Lage der Zwischenschicht ausschließlich aus chemisch vernetzten Cellulosefasern zu bilden. Die positive Wirkung der nach der Erfindung konstruierten zweilagigen Zwischenschicht resultiert aus einem synergistischen Effekt, der sich folgendermaßen erklären lässt: Durch den Gradienten des durchschnittlichen Porenvolumens (betrachtet in Richtung senkrecht zur Ebene des Deckblatts in Richtung auf den Saugkörper) wird erreicht, dass für die auf die obere Lage der Zwischenschicht auftreffende Flüssigkeit, zunächst ein verhältnismäßig großporiges Flüssigkeitsaufnahmevolumen zur Verfügung steht und dass die Flüssigkeit dann über die untere Lage der Zwischenschicht in den Saugkörper gelangen kann. Die geringere Hydrophilität der synthetischen Fasern der oberen Lage der Zwischenschicht gegenüber den chemisch vernetzten Cellulosefasern der unteren Lage der Zwischenschicht und wiederum deren vorzugsweise geringere Hydrophilität gegenüber den nichtvernetzten Cellulosefasern des Saugkörpers unterstützen die gewünschte Richtung des Flüssigkeitstransports. Die aus den chemisch vernetzten Cellulosefasern bestehende untere Lage der Zwischenschicht weist aufgrund der kapillaren Saugwirkung der geschichteten Fasern ein gewisses Flüssigkeitsleitvermögen auf, so dass durch die obere Lage der Zwischenschicht in die untere Lage gelangte Flüssigkeit in deren Ebene verteilt und somit zum Teil auch in vom Flüssigkeitsanfall beabstandete Bereiche gelangt und dort in den darunter angeordneten Saugkörper abgeführt werden kann. Dennoch wird nach mehrmaligem Auftreffen von Flüssigkeit die Speicherkapazität des Saugkörpers in dem Bereich, in dem die Flüssigkeit auftrifft, nahezu erschöpft sein. In diesem für das Funktionieren des Hygieneartikels kritischen Moment kann die untere Lage der Zwischenspeicherschicht die weiter hinzukommende Flüssigkeit zumindest in beschränktem Ausmaß speichern. Die Speicherkapazität wird zum einen aufgrund des noch vorhandenen molekularen Bindevermögens der vernetzten Fasern und zum anderen durch das zwischen den Fasern verbliebene Porenvolumen erreicht. Die untere Lage der Zwischenschicht bietet somit eine Art Reservespeicherkapazität. Die Gefahr des Herauspressens der dort gespeicherten Flüssigkeit an die Windeloberfläche, d.h. an die Haut des Benutzers der Windel, ist daher wesentlich reduziert, da die obere großporige und mit einer noch geringeren Hydrophilität der synthetischen Fasern ausgestattete Lage der Zwischenspeicherschicht als Abstandshalter fungiert. Die synthetischen Fasern der oberen Lage der Zwischenschicht bieten im bevorzugten Fall sehr geringer Hydrophilität kaum Möglichkeit zur molekularen Bindung von Flüssigkeit, so dass diese Schicht weitestgehend trocken bleibt.

Dadurch, dass die obere Lage der Zwischenschicht mit ihren Längsendabschnitten die Längsenden der unteren Lage der Zwischenschicht überlappt, lässt sich sicher vermeiden, dass eventuell in der unteren Lage der Zwischenschicht vorhandene Flüssigkeit an die Oberfläche des Hygieneartikels gedrückt werden kann.

Es hat sich desweiteren als vorteilhaft erwiesen, wenn die Ränder der Längsendabschnitte der oberen Lage noch innerhalb d.h. in einem Abstand von den Längsenden des Saugkörpers liegen. Hierdurch lässt sich die Speicherkapazität des Saugkörpers besser ausnutzen: Wenn im oben beschriebenen Fall des mehrfachen Flüssigkeitsanfalls die Speicherkapazität des Saugkörpers im Auftreffbereich erschöpft ist und die untere Lage der Zwischenspeicherschicht bereits ihre Reservespeicherfunktion aufnimmt und weiter auftreffende Flüssigkeit an der Grenze zwischen den beiden Lagen der Zwischenspeicherschicht bzw. im unteren Bereich der oberen Lage der Zwischenspeicherschicht transportiert wird, so kann die Flüssigkeit bei Erreichen der Längsenden der unteren Lage der Zwischenschicht sicher in den Saugkörper eingeleitet werden, wobei davon ausgegangen wird, dass die Speicherkapazität des Saugkörpers in diesem Bereich noch nicht ausgeschöpft ist.

Der vorstehend erwähnte Flüssigkeitstransport im unteren Bereich der oberen Lage der Zwischenschicht lässt sich noch effektiver gestalten, wenn gemäß einer bevorzugten Ausführungsform die obere Lage ihrerseits ein in Richtung des Flüssigkeitstransports vom Deckblatt zum Saugkörper, also senkrecht zur Schichtebene abnehmendes Porenvolumen aufweist. Das Porenvolumen braucht nicht diskret von einem größeren Wert zu einem kleineren Wert abzunehmen, sondern es kann quasi kontinuierlich innerhalb der oberen Lage der Zwischenschicht von oben nach unten abnehmen.

Bevorzugtermaßen wird die Zwischenschicht so gebildet, dass die Fasern der oberen Lage der Zwischenschicht in die untere Lage der Zwischenschicht eingreifen und somit eine Übergangszone zwischen den Lagen gebildet wird. In entsprechender Weise können die Fasern der unteren Lage der Zwischenschicht in den Saugkörper eingreifen, so dass ebenfalls eine Übergangszone zwischen der Zwischenschicht und dem Saugkörper gebildet wird.

Durch den vorstehend beschriebenen zweischichtigen Aufbau der Zwischenschicht und der Funktionsweise der unteren Lage der Zwischenschicht ist es möglich, das Flächengewicht der oberen Lage der Zwischenschicht gering zu halten, vorzugsweise unterhalb von 55 g/m². Dies eröffnet aber die Möglichkeit, eine Endlosrolle des hierfür benötigten Materials mit einer für den technischen Herstellungsvorgang befriedigend hohen Lauflänge auszustatten und das Material in die schnelllaufende Fertigung zu integrieren.

Zur Bildung der oberen Lage der Zwischenschicht wird vorgeschlagen, einen im Vergleich zum Vliesstoff des Deckblatts voluminösen Vliesstoff zu verwenden. Dem Fachmann stehen eine Reihe von bekannten synthetischen Faserarten sowie Verfahren zur Vliesstoffherstellung zur Verfügung, so dass dies keiner näheren Beschreibung bedarf. Bevorzugt wird der Vliesstoff zumindest anteilig aus ansich bekannten Bikomponentenfasern, z.B. des Kern/Mantel-Typs gebildet. Damit lässt sich ein Bikomponentenfasern enthaltendes Faservlies in bekannter Weise thermisch zum fertigen Vliesstoff konsolidieren, ohne dass die Faserstruktur beeinträchtigt wird. Als vorteilhaft haben sich Faserstärken der Bikomponentenfasern von wenigstens 4 dtex, vorzugsweise von 5 bis 8 dtex erwiesen. Hierdurch wird gewährleistet, dass der Titer der Fasern auch nach der thermischen Konsolidierung noch groß genug ist, um dem Vliesstoff das gewünschte große Porenvolumen zu geben. Dicke Fasern lassen sich nämlich nicht so volumenfüllend packen wie dünne Fasern. Dem Fachmann stehen desweiteren Mittel zur Einstellung der Hydrophilität von synthetischen Fasern zur Verfügung, die daher keiner Erläuterung bedürfen. Unter Verwendung derartiger Mittel lässt sich die vorzugsweise sehr geringe Hydrophilität der oberen Lage der Zwischenschicht so einstellen, dass sie vorzugsweise sehr gering in jedem Fall jedoch geringer als diejenige der chemisch vernetzten Fasern der unteren Lage der Zwischenschicht ist. Es hat sich auch bewährt, den unterhalb der Zwischenschicht angeordneten Saugkörper zweilagig auszubilden, wobei die obere Lage im wesentlichen Cellulosefasern und superabsorbierende Polymer-Materialien und die untere Lage im wesentlichen ausschließlich Cellulosefasern enthält. Hierdurch wird eine optimale Ausnutzung der Saugkapazität des Saugkörpers gewährleistet.

Weitere Vorteile, Einzelheiten und Merkmale der Erfindung ergeben sich aus den beigefügten Patentansprüchen und der beigefügten zeichnerischen Darstellung und nachfolgenden Beschreibung einer bevorzugten Ausführungsform der Erfindung. In der Zeichnung zeigt:
- Figur 1: eine schematische Darstellung des Aufbaus eines erfindungsgemäßen Hygieneartikels und
- Figur 2: eine Längsschnittansicht der durch die Pfeile II-II bezeichneten Ebene.

Die Figuren zeigen in schematischer Darstellung den Aufbau eines Hygieneartikels in Form einer Windel. Die Windel umfasst ein Deckblatt 2, ein flüssigkeitsundurchlässigen Rückenblatt 4 und einen dazwischen angeordneten Saugkörper 6. Zwischen dem Deckblatt 2 und dem Saugkörper 6 ist eine zweischichtige insgesamt mit dem Bezugszeichen 8 bezeichnete Zwischenschicht vorgesehen. Im dargestellten Fall erstreckt sich das Deckblatt 2 in Längsrichtung 10 der Windel bis zu deren Längsrändern 12 und in Querrichtung 14 bis zu den seitlichen Querrändern 16 der Windel. Im Bereich entlang des Verlaufs der Längsränder 12 und Querränder 16 ist das flüssigkeitsdurchlässige Deckblatt 2 mit dem Rückenblatt 4 durch ansich beliebige und bekannte Fügeverfahren verbunden. Es wird darauf hingewiesen, dass auch eine Ausführungsform denkbar wäre, bei der das Deckblatt 2 in einem Abstand zu den Querrändern 16 endet und dass außerhalb des Deckblatts 2 weitere bspw. bündchenbildende Elemente oder Elastifizierungsmittel vorgesehen werden können.

Die Zwischenschicht 8 ist zweilagig ausgebildet und weist eine obere Lage 18 und eine untere Lage 20 auf. Die obere Lage 18 überlappt mit Längsendabschnitten 19 in Längsrichtung 10 die Längsenden 21 der unteren Lage 20, sie endet aber noch innerhalb bzw. in einem Abstand von Längsenden 22 des Saugkörpers 6. In Querrichtung 14 haben die obere und die untere Lage 18 bzw. 20 dieselbe Breite. Es wäre aber auch denkbar und vorteilhaft, dass die obere Lage 18 die untere Lage 20 auch in Querrichtung überlappt.

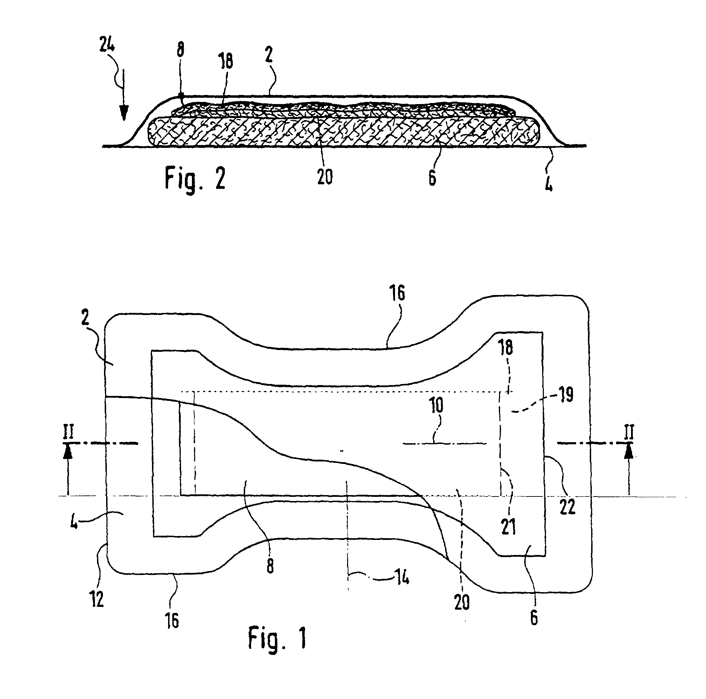

Die obere Lage 18 ist von einem aus synthetischen Fasern bestehenden Vliesstoff gebildet, wobei bevorzugtermaßen ein Anteil von Bikomponentenfasern zum Einsatz kommen. Der Vliesstoff ist im Zuge seiner Herstellung derart verdichtet, dass die obere Lage 18 in Richtung des Pfeils 24 in Figur 2, d.h. in Richtung des Flüssigkeitstransports senkrecht zur flächigen Erstreckung der Zwischenschicht 8, ein abnehmendes Porenvolumen aufweist.

Die untere Lage 20 besteht aus chemisch vernetzten Cellulosefasern, wobei das Porenvolumen der unteren Lage 20 geringer ist als dasjenige der oberen Lage 18 der Zwischenschicht 8. Auch ist die Hydrophilität der chemisch vernetzten Cellulosefasern der unteren Lage 20 größer als diejenige der synthetischen Fasern des die obere Lage 18 bildenden Vliesstoffs.

Bei starkem Flüssigkeitsanfall, wenn also das Flüssigkeitsaufnahmevermögen des Saugkörpers 6 im Bereich des Flüssigkeitsanfalls bereits erschöpft ist, übernimmt die Zwischenschicht 8 eine Flüssigkeitszwischenspeicher und leitfunktion. Zunächst wird erneut schwallartig anfallende Flüssigkeit in dem verhältnismäßig großporigen Volumen der oberen Lage 18 sehr kurzfristig aufgenommen und dann an die untere Lage 20 der Zwischenschicht 8 abgegeben, womit diese ihre Reservespeicherfunktion ausübt. Ist auch die Speicherkapazität der unteren Lage 20 erschöpft, findet im Bereich des Übergangs der oberen Lage 18 zur unteren Lage 20 eine Flüssigkeitsleitung in der Ebene der flächenhaft erstreckten Lagen statt. Hierdurch wird Flüssigkeit vom Ort der Einleitung wegtransportiert und in im Hinblick auf die Flüssigkeitsaufnahmekapazität noch nicht erschöpfte Bereiche des Saugkörpers 6 in diesen eingeleitet.

## Patentansprüche

1. Absorbierender Hygieneartikel zum einmaligen Gebrauch mit einem flüssigkeitsdichten Rückblatt (4), einem flüssigkeitsdurchlässigen Deckblatt (2), einem dazwischen angeordneten zur Speicherung von Körperflüssigkeiten, insbesondere Urin, geeigneten superabsorbierende Polymer-Materialien enthaltenden Saugkörper (6) und einer zweilagigen flüssigkeitsaufnehmenden und -verteilenden Zwischenschicht (8), die aus einer oberen Lage (18) aus synthetischen Fasern und einer unteren Lage (20) besteht, wobei das durchschnittliche Porenvolumen der oberen Lage (18) größer ist als das durchschnittliche Porenvolumen der unteren Lage (20) der Zwischenschicht (8) und wobei die Zwischenschicht zwischen dem Saugkörper (6) und dem Deckblatt (2) angeordnet ist, **dadurch gekennzeichnet, dass** die untere Lage (20) aus chemisch vernetzten Cellulosefasern gebildet ist und die obere Lage (18) mit ihren Längsendabschnitten (19) die Längsenden (21) der unteren Lage (20) überlappt und dass die Hydrophilität der chemisch vernetzten Cellulosefasern der unteren Lage (20) größer ist als die der synthetischen Fasern der oberen Lage (18) der Zwischenschicht (8).

2. Hygieneartikel nach Anspruch 1, **dadurch gekennzeichnet, dass** die Längsenden der oberen Lage (18) der Zwischenschicht (8) von den Längsenden (22) des Saugkörpers beabstandet sind.

3. Hygieneartikel nach einem der vorstehenden Ansprüche, **dadurch gekennzeichnet, dass** die obere Lage (18) durch einen Bikomponentenfasern enthaltenden Vliesstoff gebildet ist.

4. Hygieneartikel nach Anspruch 3, **dadurch gekennzeichnet, dass** die Bikomponentenfasern eine Faserstärke von mindestens 4 dtex, vorzugsweise von 5 bis 8 dtex, aufweisen.

5. Hygieneartikel nach einem der vorstehenden Ansprüche, **dadurch gekennzeichnet, dass** die flächenbezogene Masse des die obere Lage (18) der Zwischenschicht (8) bildenden Vliesstoffs kleiner als 55 g/m² ist.

6. Hygieneartikel nach einem der vorstehenden Ansprüche, **dadurch gekennzeichnet, dass** die aus chemisch vernetzten Cellulosefasern gebildete untere Lage (20) der Zwischenschicht (8) frei von SAP-Materialien ist.

7. Hygieneartikel nach einem der vorstehenden Ansprüche, **dadurch gekennzeichnet, dass** die durchschnittliche Porengröße der unteren Lage (20) der Zwischenschicht (8) größer ist als die des Saugkörpers (6).

8. Hygieneartikel nach einem der vorstehenden Ansprüche, **dadurch gekennzeichnet, dass** das Porenvolumen der oberen Lage (18) der Zwischenschicht (8) in Richtung senkrecht zur unteren Lage (20) abnimmt.

9. Hygieneartikel nach einem der vorstehenden Ansprüche, **dadurch gekennzeichnet, dass** die obere Lage (18) der Zwischenschicht (8) eine obere Teilschicht und eine untere Teilschicht aufweist, wobei das Porenvolumen der oberen Teilschicht größer ist als das der unteren Teilschicht.

10. Hygieneartikel nach einem der vorstehenden Ansprüche, **dadurch gekennzeichnet, dass** die Fasern der oberen Lage (18) der Zwischenschicht (8) in die untere Lage (20) der Zwischenschicht (8) eingreifen, so dass eine Übergangszone zwischen den Lagen (18, 20) gebildet.

11. Hygieneartikel nach einem der vorstehenden Ansprüche, **dadurch gekennzeichnet, dass** die Fasern der unteren Lage (20) der Zwischenschicht (8) in den Saugkörper (6) eingreifen, so dass eine Übergangszone zwischen der Zwischenschicht (8) und dem Saugkörper (6) gebildet wird.

12. Hygieneartikel nach einem der vorstehenden Ansprüche, **dadurch gekennzeichnet, dass** der Saugkörper zweilagig ist, wobei die obere Lage im wesentlichen Cellulosefasern und superabsorbierendes Polymer enthält und die untere Lage im wesentlichen ausschließlich Cellulosefasern enthält.

## Claims

1. Absorbent disposable sanitary article with a liquid-impervious backing sheet (4), a liquid-permeable outer sheet (2), an absorbent member (6) arranged therebetween to store bodily fluids, in particular urine, which contains suitable super-absorbent polymer materials, and a two-ply liquid-absorbing and -distributing intermediate layer (8) which consists of an upper ply (18) made of synthetic fibres and a lower ply (20), the mean pore volume of the upper ply (18) being greater than the mean pore volume of the lower ply (20) of the intermediate layer (8) and the intermediate layer being arranged between the absorbent member (6) and the outer sheet (2), **characterised in that** the lower ply (20) is formed from chemically crosslinked cellulose fibres and the upper ply (18) overlaps with its longitudinal end portions (19) the longitudinal ends (21) of the lower ply (20), and **in that** the hydrophilicity of the chemically crosslinked cellulose fibres of the lower ply (20) is greater than that of the synthetic fibres of the upper ply (18) of the intermediate layer (8).

2. Sanitary article according to claim 1, **characterised in that** the longitudinal ends of the upper ply (18) of the intermediate layer (8) are spaced apart from the longitudinal ends (22) of the absorbent member.

3. Sanitary article according to any of the preceding claims, **characterised in that** the upper ply (18) is formed by a non-woven material which contains bi-component fibres.

4. Sanitary article according to claim 3, **characterised in that** the bi-component fibres have a fibre thickness of at least 4 dtex, preferably of 5 to 8 dtex.

5. Sanitary article according to any of the preceding claims, **characterised in that** the mass by area of the non-woven material forming the upper ply (18) of the intermediate layer (8) is less than 55 g/m².

6. Sanitary article according to any of the preceding claims, **characterised in that** the lower ply (20) of the intermediate layer (8) formed from chemically crosslinked cellulose fibres is free of SAP materials.

7. Sanitary article according to any of the preceding claims, **characterised in that** the mean pore size of the lower ply (20) of the intermediate layer (8) is greater than that of the absorbent member (6).

8. Sanitary article according to any of the preceding claims, **characterised in that** the pore volume of the upper ply (18) of the intermediate layer (8) decreases in the direction perpendicular to the lower ply (20).

9. Sanitary article according to any of the preceding claims, **characterised in that** the upper ply (18) of the intermediate layer (8) has an upper partial layer and a lower partial layer, the pore volume of the upper partial layer being greater than that of the lower partial layer.

10. Sanitary article according to any of the preceding claims, **characterised in that** the fibres of the upper ply (18) of the intermediate layer (8) mesh in the lower ply (20) of the intermediate layer (8) so a transition zone is formed between the layers (18, 20).

11. Sanitary article according to any of the preceding claims, **characterised in that** the fibres of the lower ply (20) of the intermediate layer (8) mesh in the absorbent member (6), so a transition zone is formed between the intermediate layer (8) and the absorbent member (6).

12. Sanitary article according to any of the preceding claims, **characterised in that** the absorbent member is two-ply, the upper ply substantially containing cellulose fibres and super-absorbent polymer and the lower ply substantially containing cellulose fibres exclusively.

## Revendications

1. Article hygiénique absorbant à usage unique, doté d'un feuillet inférieur étanche aux liquides (4), d'un feuillet supérieur perméable aux liquides (2), d'un coussin absorbant (6) adapté contenant des polymères superabsorbants et agencé entre ces deux feuillets pour retenir des liquides corporels, en particulier de l'urine, et d'un matelas intermédiaire (8) à deux couches pour capter et répartir le liquide, ledit matelas intermédiaire se composant d'une couche supérieure (18) en fibres synthétiques et d'une couche inférieure (20), le volume poreux moyen de la couche supérieure (18) étant supérieur à celui de la couche inférieure (20) du matelas intermédiaire (8) et le matelas intermédiaire étant agencé entre le coussin absorbant (6) et le feuillet supérieur (2), **caractérisé en ce que** la couche inférieure (20) est composée de fibres cellulosiques chimiquement réticulées, **en ce que** les zones terminales longitudinales (19) de la couche supérieure (18) recouvrent les extrémités longitudinales (21) de la couche inférieure (20) et **en ce que** l'hydrophilie des fibres cellulosiques chimiquement réticulées de la couche inférieure (20) est supérieure à celle des fibres synthétiques de la couche supérieure (18) du matelas intermédiaire (8).

2. Article hygiénique selon la revendication 1, **caractérisé en ce que** les extrémités longitudinales de la couche supérieure (18) du matelas intermédiaire (8) sont distantes des extrémités longitudinales (22) du coussin absorbant.

3. Article hygiénique selon l'une des revendications précédentes, **caractérisé en ce que** la couche supérieure (18) est constituée d'un non-tissé comportant des fibres bicomposant.

4. Article hygiénique selon la revendication 3, **caractérisé en ce que** les fibres bicomposant ont un titre supérieur ou égal à 4 dtex et de préférence compris entre 5 et 8 dtex.

5. Article hygiénique selon l'une quelconque des revendications précédentes, **caractérisé en ce que** la masse surfacique du non-tissé formant la couche supérieure (18) du matelas intermédiaire (8) est inférieure à 55 g/m².

6. Article hygiénique selon l'une des revendications précédentes, **caractérisé en ce que** la couche inférieure (20) formée de fibres cellulosiques chimiquement réticulées ne contient pas de polymères superabsorbants.

7. Article hygiénique selon l'une des revendications précédentes, **caractérisé en ce que** la taille moyenne des pores de la couche inférieure (20) du matelas intermédiaire (8) est supérieure à celle du coussin absorbant (6).

8. Article hygiénique selon l'une des revendications précédentes, **caractérisé en ce que** le volume poreux de la couche supérieure (18) du matelas intermédiaire (8) décroît verticalement en direction de la couche inférieure (20).

9. Article hygiénique selon l'une des revendications précédentes, **caractérisé en ce que** la couche supérieure (18) du matelas intermédiaire (8) présente une couche partielle supérieure et une couche partielle inférieure, le volume poreux de la couche partielle supérieure étant supérieur à celui de la couche partielle inférieure.

10. Article hygiénique selon l'une des revendications précédentes, **caractérisé en ce que** les fibres de la couche supérieure (18) du matelas intermédiaire (8) s'engagent dans la couche inférieure (20) du matelas intermédiaire (8), formant une zone de transition entre les couches (18, 20).

11. Article hygiénique selon l'une des revendications précédentes, **caractérisé en ce que** les fibres de la couche inférieure (20) du matelas intermédiaire (8) s'engagent dans le coussin absorbant (6), formant une zone de transition entre le matelas intermédiaire (8) et le coussin absorbant (6).

12. Article hygiénique selon l'une des revendications précédentes, **caractérisé en ce que** le coussin absorbant est composé de deux couches, la couche supérieure comportant essentiellement des fibres cellulosiques et un polymère superabsorbant et la couche inférieure contenant quasi exclusivement des fibres cellulosiques.
